# EUROPEAN PATENT APPLICATION

(11) **EP 3 479 716 A1**
(43) Date of publication of application: **08.05.2019**
(21) Application number: 16906702.2
(22) Date of filing: 30.06.2016
(51) Int. Cl.: A43B 5/00, G01C 22/00

(54) **PEDOMETER SHOE**

(71) Applicant: Shenzhen Royole Technologies Co., Ltd., Shenzhen, Guangdong 518052 (CN)
(72) Inventor: LIU, Wenjun, Shenzhen Guangdong 518052 (CN); XIA, Xinyuan, Shenzhen Guangdong 518052 (CN)
(74) Representative: Hamer, Christopher K.
(86) International application number: PCT/CN2016/087843
(87) International publication number: WO 2018/000301

(57) **Abstract**

A pedometer shoe (100), includes: a shoe body (1) including a vamp (11) and a shoe sole (12) which are connected with each other; a control module (2) which is arranged in the shoe sole (12) and sends out signals according to step counts; and a display module (3) which is attached to the vamp (11), the display module (3) including a display area (30) and receiving the signals and displaying corresponding prompts. The pedometer shoe (100) may count steps and display the step count.

## Description

### TECHNICAL FIELD

The present disclosure relates to the technical field of pedometer device, and more particularly relates to a pedometer shoe.

### BACKGROUND

In modern society, people pay more and more attention to health. Many people make daily exercise plans for themselves. Walking and running are the most popular ways of exercise.

In order to measure the exercise amount, the industry has developed pedometer shoes. In existing pedometer shoes, a pedometer is usually provided on a vamp or a shoe sole, a motion step count is counted by the pedometer, and the step count is sent to a software (for example, an app in a mobile phone) in a mobile terminal, and finally the motion step count is displayed by the mobile terminal. However, it is usually inconvenient for people to carry the mobile terminals during exercise, so it is impossible for people to know about the motion step count intuitively and in time during their exercise, which makes the pedometer shoes be not as popular as expected.

### SUMMARY

The technical problem to be solved in the present disclosure is to provide a pedometer shoe which is capable of counting steps and displaying a step count.

In order to achieve the above object, the following technical solutions are provided in embodiments of the present disclosure.

A pedometer shoe is provided, which includes a shoe body, a control module, and a display module. The shoe body includes a vamp and a shoe sole which are connected with each other. The control module is arranged in the shoe sole and sends out signals according to step counts. The display module is attached to the vamp, the display module includes a display area, and receives the signals and displays corresponding prompts.

Compared with related art, the present disclosure has the following beneficial effects.

According to the pedometer shoe of the embodiments of the present disclosure, since the control module may send out signals according to the step counts, and the display module may receive the signals and display corresponding prompts, that is, the pedometer shoe may count steps and display a step count, so that the user can learn his or her real-time exercise amount in time.

### BRIEF DESCRIPTION OF THE DRAWINGS

To better illustrate the technical solutions of the present disclosure, the following descriptions will briefly illustrate the accompanying drawings described in the embodiments. Obviously, the following described accompanying drawings are merely some embodiments of the present disclosure. Those of ordinary skill in the art can obtain other accompanying drawings according to the described accompanying drawings without creative efforts.
FIG. 1 is a schematic structural diagram of a pedometer shoe according to an embodiment of the present disclosure.
FIG. 2 is a schematic diagram of the pedometer shoe viewed from another perspectives according to an embodiment of the present disclosure.
FIG. 3 is a schematic structural diagram of a display module and a control module of the pedometer shoe according to an embodiment of the present disclosure.
FIG. 4 is a schematic exploded view of the display module and the control module of the pedometer shoe according to an embodiment of the present disclosure.
FIG. 5 is a schematic exploded view of the control module of the pedometer shoe according to an embodiment of the present disclosure.
FIG. 6 is a schematic exploded view of the control module of the pedometer shoe viewed from another perspectives according to an embodiment of the present disclosure.
FIG. 7 is a schematic structural diagram of a shoe sole of the pedometer shoe according to an embodiment of the present disclosure.

### DETAILED DESCRIPTION OF ILLUSTRATED EMBODIMENTS

The technical solutions of embodiments of the present disclosure will be described clearly and completely in the following in combination with the accompanying drawings of the embodiments of the present disclosure. Obviously, the described embodiments are merely a part of embodiments of the present disclosure, but not all of the embodiments. All other embodiments obtained by those of ordinary skill in the art without creative efforts based on the embodiments of the present disclosure shall fall within the protection scope of the present disclosure.

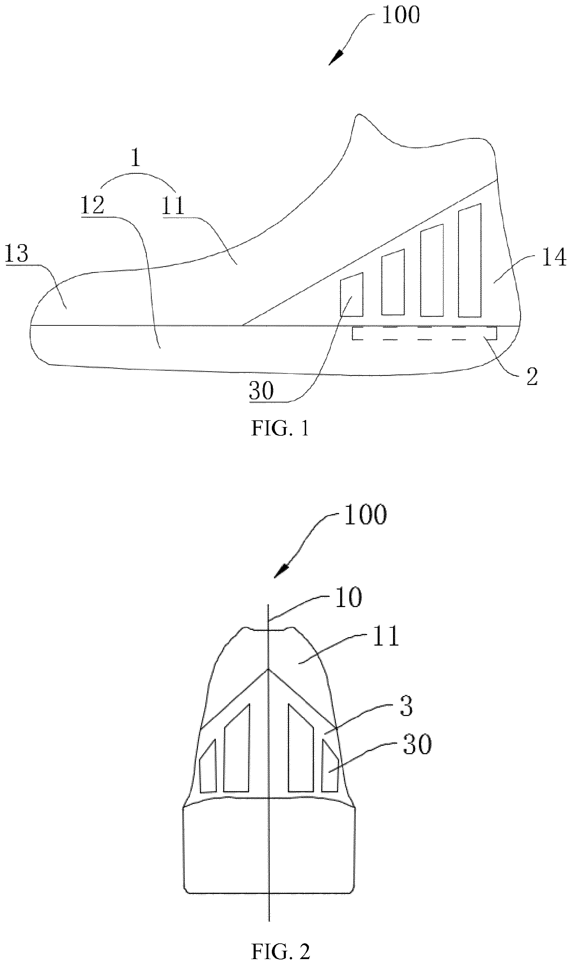

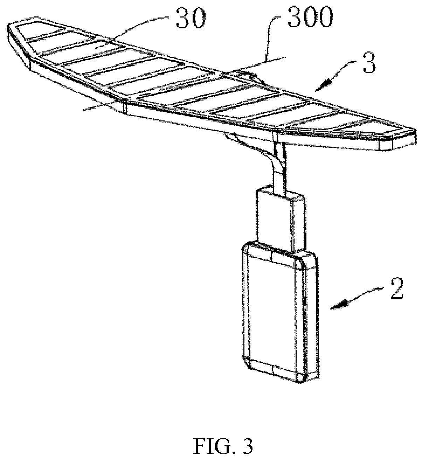

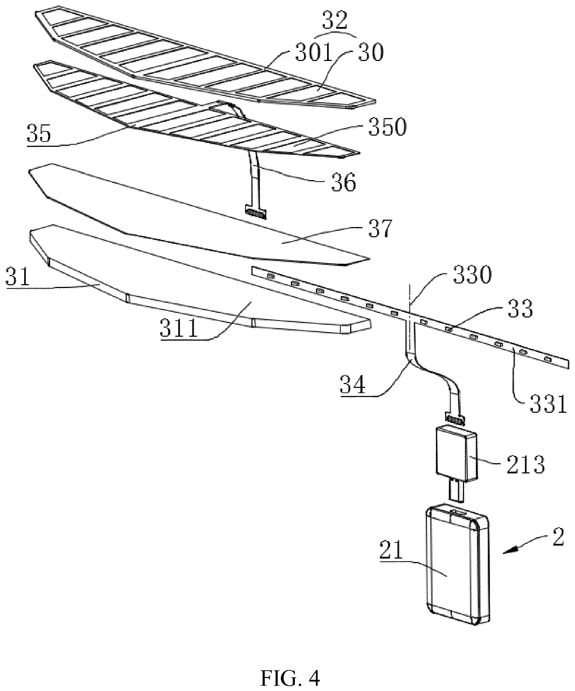

Referring to FIGs. 1 to 4, the embodiments of the present disclosure provide a pedometer shoe 100, which includes a shoe body 1, a control module 2 and a display module 3. The shoe body 1 includes a vamp 11 and a shoe sole 12 which are connected with each other. The vamp 12 includes a shoe toe 13 corresponding to a toe position and a shoe upper 14 corresponding to a heel position. The control module 2 is arranged in the shoe sole 12 and sends out signals according to step counts. The display module 3 is attached to the vamp 11. The display module 3 includes a display area 30, and receives the signals and displays corresponding prompts.

In the embodiment, since the control module 2 may send out signals according to the step counts, and the display module 3 may receive the signals and display corresponding prompts, that is, the pedometer shoe 100 may count steps and display the step counts, so that a user can learn his or her real-time exercise amount in time.

It should be understood that, the prompts are displayed in the display area 30. Preferably, since the shoe upper 14 has a large and flat surface, the display module 3 may be attached to the shoe upper 14, so as to enhance the overall appearance of the pedometer shoe 100.

Further, as an alternative embodiment, the display module 3 may display different colors according to different step counts. For example, the display module 3 displays at least two colors. When the step count reaches 2500 steps, the display area 30 presents red; when the step count reaches 5000 steps, the display area 30 presents yellow; when the step count reaches 7500 steps, the display area 30 presents green, etc.

In the embodiment, since the colors displayed in the display area 30 are different, the user may intuitively determine the step count represented by the prompt, and the prompt is beautiful and diverse, which is beneficial to improve the user's experience of the pedometer shoe 100.

Further, as an alternative embodiment, the display module 3 includes a plurality of the display areas 30, and the display areas 30 are lighted up one by one according to different step counts. For example, when the step count reaches 2500 steps, one of the display areas 30 is lighted up; when the step count reaches 5000 steps, two of the display areas 30 are lighted up; when the step count reaches 7500 steps, three of the display areas 30 are lighted up; when the step count reaches 10000 steps, four of the display areas 30 are lighted up, etc.

In the embodiment, since the numbers of the lighted up display areas 30 are different, the user may intuitively know about the step count represented by the prompt, and the prompt is beautiful and diverse, which is beneficial to improve the user's experience of the pedometer shoe 100.

Further, as an alternative embodiment, heights of the plurality of display areas 30 arranged from the shoe upper 14 toward the shoe toe 13 of the vamp 11 are gradually decreased. In other words, a direction along the shoe upper 14 toward the shoe toe 13 is defined as a first direction, and the heights of the plurality of display areas 30 arranged along the first direction are gradually decreased. The display areas 30 with gradually decreased heights may be configured to represent a range of steps in which the values are gradually decreased, to form an intuitive and obvious visual sense.

For example, the plurality of display areas 30 include a first display area, a second display area, a third display area, and a fourth display area, which have sequentially increased heights.

When the step count reaches 2500 steps, the first display area is lighted up; when the step count reaches 5000 steps, the second display area is lighted up; when the step count reaches 7500 steps, the third display area is lighted up; and when the step count reaches 10000 steps, the fourth display area is lighted up.

Alternatively, when the step count reaches 2500 steps, the first display area is lighted up; when the step count reaches 5000 steps, the first display area and the second display area are lighted up; when the step count reaches 7500 steps, the first display area, the second display area and the third display area are lighted up; and when the step count reaches 10000 steps, the first display area, the second display area, the third display area and the fourth display area are lighted up.

In the embodiment, the increasing of the display height and/or the display number of the display areas enables the user to intuitively feel the increasing of the motion steps, which is beautiful and direct, and improves the user's experience.

It should be understood that, the display pattern of the prompt displayed by the display module 3 may be derived from a combination of the plurality of display areas 30, and obvious difference between a plurality of different display patterns formed by different combinations, enables the user to intuitively determine the step count represented by the display pattern. Moreover, the combined display patterns are beautiful and diverse, which is beneficial to improve the user's experience of the pedometer shoe 100.

The "different combinations" include, but are not limited to, different colors displayed by the display area 30, different heights of the display areas 30, different numbers of the display areas 30 for display, different arrangements of the display area 30 for display, or different combinations of the above means, etc.

Further, referring to FIGs. 1 to 4, as an alternative embodiment, the display module 3 includes a light guide plate 31 and a light source 33. The light guide plate 31 includes a light exiting surface 311 and a reflecting surface which are opposite to each other, and a light incident surface connected between the light exiting surface 311 and the reflecting surface. The reflecting surface is arranged facing an inside of the shoe body 1, and the light exiting surface 311 is arranged facing an outside of the shoe body 1, so that light can exit from the shoe body 1, to realize the display function of the display module 3. The light source 33 faces the light incident surface, and light emitted from the light source 33 passes through the light incident surface and exits from the light exiting surface 311. That is, the light emitted from the light source 33 enters the light guide plate 31 from the light incident surface, and exits from the light exiting surface 311 and enters human eye after being reflected by the reflecting surface, to realize the display of the display module 3.

Preferably, a plurality of the light sources 33 are provided. The light sources 33 are arranged in one-to-one correspondence with the plurality of display areas 30, that is, each display area 30 corresponds to a light source 33. Since each display area 30 corresponds to a light source 33, the combinations of the plurality of display areas 30 can be controlled by controlling the states of the plurality of light sources 33. The combination process is flexible and variable, so that the prompts of the display module 3 can be rendered in a variety of different display patterns.

Further, as an alternative embodiment, the light guide plate 31 is made of flexible material, so that the display module 3 can be well attached to the vamp 11. Preferably, the reflecting surface of the light guide plate 31 may be attached to the vamp 11 by heat pressing or bonding, such that the plurality of display areas 30 are erected, and the light exiting direction is toward the outside of the shoe body 1.

Further, referring to FIGs. 1 to 4, as an alternative embodiment, the display module 3 further includes a flexible touch panel 35. The flexible touch panel 35 generates a touch signal according to a touch operation, and the control module 2 adjusts the prompt displayed by the display module 3 according to the touch signal.

In the embodiment, since the pedometer shoe 100 is integrated with the flexible touch panel 35, and the flexible touch panel 35 is capable of generating the touch signal according to the touch operation, and the control module 2 adjusts the prompt displayed by the display module 3 according to the touch signal. Therefore, the pedometer shoe 100 has a touch display function, and the pedometer shoe 100 is multifunctional.

Further, as an alternative embodiment, the flexible touch panel 35 can generate the touch signal according to a long-pressing touch operation, and the control module 2 adjusts the color of the display module 3 according to the touch signal, that is, adjusts the display color of the prompt.

Further, as an alternative embodiment, the flexible touch panel 35 generates the touch signal according to a sliding touch operation, and the control module 2 adjusts brightness of the display module 3 according to the touch signal, that is, adjusts the display the brightness of the prompt. The sliding operation includes, but is not limited to, sliding up and down, sliding left and right, or tilting sliding.

Further, as an alternative embodiment, the flexible touch panel 35 generates the touch signal according to a double-click touch operation, and the control module 2 turns on or off the prompt of the display module 3 according to the touch signal.

Further, referring to FIGs. 1 to 4, as an alternative embodiment, the flexible touch panel 35 is attached to the light exiting surface 311 of the light guide plate 31. The flexible touch panel 35 is made of flexible transparent material, and outputs the touch signal by sensing an external touch.

Further, referring to FIGs. 1 to 4, as an alternative embodiment, the flexible touch panel 35 includes a touch area 350 and a non-touch response area, the touch area 350 is arranged opposite to the display area 30, and the non-touch response area is located outside a range in which the touch area 350 is located. The touch area 350 is capable of outputting the touch signal by sensing an external touch.

Preferably, the flexible touch panel 35 is a single continuous flexible touch material with an entire surface can generate the touch signal in response to the touch operation. However, since the flexible touch panel 35 in the embodiment only needs to make a touch response at the position where the display area 30 is located, and does not need to make any response at the connection positions among the display areas 30, the flexible touch panel 35 thus form a plurality of non-touch response areas at the connection positions among the display areas 30. Each non-touch response area is located between two adjacent touch areas 350. The touch areas 350 are separated from each other by the non-touch response area. The non-touch response area can be made to not respond to touch operations by means of software masking.

Preferably, the flexible touch panel 35 includes a plurality of the touch areas 350 and a plurality of the non-touch response areas, and the touch areas 350 and the non-touch response areas are alternately arranged. Such that, the plurality of touch areas 350 are sequentially arranged at intervals, render in beautiful appearance, and respond to the touch operations accurately.

Further, referring to FIGs. 1 to 4, as an alternative embodiment, the display module 3 further includes a protective film 32 attached to the flexible touch panel 35. The flexible touch panel 35 is sandwiched between the protective film 32 and the light guide plate 31.

Preferably, the protective film 32 is attached to the flexible touch panel 35 by means of hot pressing or bonding. The protective film 32 covers the flexible touch panel 35 to protect the flexible touch panel 35.

Further, referring to FIGs. 1 to 4, as an alternative embodiment, the protective film 32 includes the display area 30 and an opaque area 301. The opaque area 301 is located outside a range in which the display area 30 is located. The display area 30 is capable of displaying the prompt, and the opaque area 301 does not display anything.

Preferably, the protective film 32 includes a plurality of the display areas 30 and a plurality of the opaque areas 301, and the opaque areas 301 and the display areas 30 are alternately arranged. Such that, the plurality of display areas 30 are sequentially arranged at intervals, so as to make the prompt beautiful and clear when displayed.

Further, referring to FIGs. 1 to 4, as an alternative embodiment, the display module 3 further includes a uniform light film 37 arranged between the flexible touch panel 35 and the light exiting surface 311, the uniform light film 37 is configured to homogenize the light exiting from the light guide plate 31. The uniform light film 37 is made of flexible light transparent material.

Further, referring to FIGs. 1 to 4, as an alternative embodiment, the display module 3 further includes a first flexible circuit board 331, and the light source 33 is mounted on the first flexible circuit board 331. That is, the first flexible circuit board 331 can be configured to hold up and be electrically connected to the light source 33. The first flexible circuit board 331 locates at the light incident surface side of the light guide plate 31. The light source 33 can be a colored light emitting diode such as an RGB-LED.

Preferably, the first flexible circuit board 331 is bent around the shoe upper 14 of the vamp 11. At this time, the whole display module 3 is bent around the shoe upper 14 of the vamp 11.

Further, referring to FIGs. 1 to 4, as an alternative embodiment, the first flexible circuit board 331 is electrically coupled to the control module 2 through a second flexible circuit board 34, and the flexible touch panel 35 is electrically coupled to the control module 2 through a third flexible circuit 36. The second flexible circuit board 34 and the third flexible circuit board 36 are freely bent according to the shape of the pedometer shoe 100.

Further, referring to FIGs. 1 to 4, as an alternative embodiment, the display module 3 is bent around the shoe upper 14 of the vamp 11 and is attached to two opposite sides of the vamp 11, to facilitate the user to view the prompt from either side of the pedometer shoe 100 (either on the left or right side).

For example, the plurality of display areas 30 include two display regions that are symmetrical, and the two display regions are respectively located at two sides of a first center line 300. Each display region includes at least two display areas 30, and the heights of the at least two display areas 30 located in the same display region are sequentially increased along a direction. That is, in a same display region, at least two height-increasing display areas 30 are included. The display areas 30 having different heights can be used to represent different ranges of steps, and the height-increasing display areas 30 can be used to represent a range of steps in which the values are incremented to form an intuitive, apparent visual sense.

It should be understood that, the two display regions are not only symmetrical in shape, but the display patterns displayed are also symmetrical. Preferably, the heights of the display areas 30 in the two display regions are increased in a direction toward each other. The height in the present embodiment refers to the length of the display area 30 in a direction paralleled to the first center line 300.

The plurality of light sources 33 are divided into two symmetrical light source groups which are respectively located at two sides of a second center line 330. Each of the light source groups includes at least two light sources 33, and the at least two light sources 33 in a same light source group have different colors. Since the light sources 33 are arranged in one-to-one correspondence with the display area 30, at least two display areas 30 having different display colors are included in each display region. Different display colors allow greater visibility of the display module 3, and more combinations of display patterns are obtained, which also makes the pedometer shoe 100 more beautiful.

The shoe upper 14 of the pedometer shoe 100 defines a dorsal midline 10, and the two display regions of the display module 3 are respectively located at two sides of the dorsal midline 10. At this time, the first center line 300 and the second center line 330 are both collinear with the dorsal midline 10, and the two light source groups are respectively located at two sides of the dorsal midline 10, to facilitate the user to view the motion steps from either side of the pedometer shoe 100 (either on the left or right side).

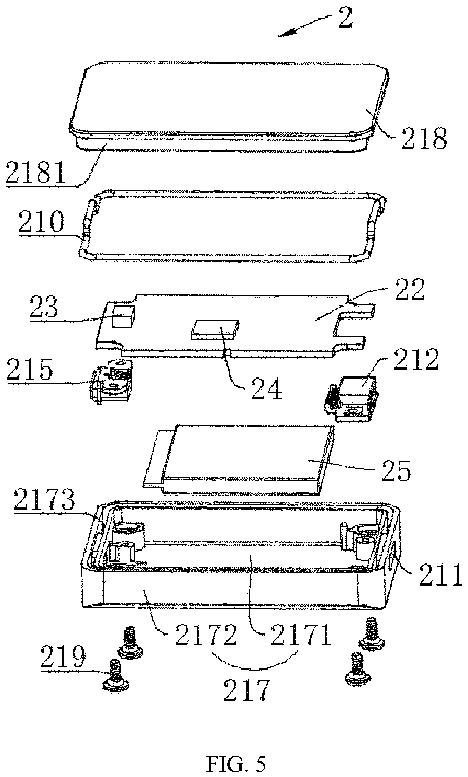

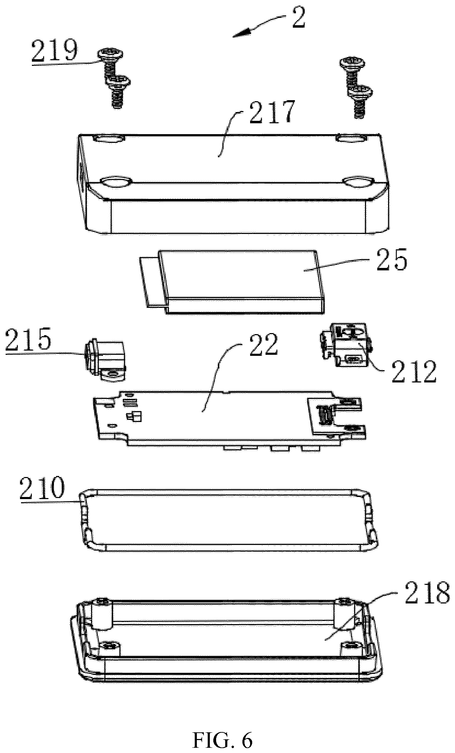

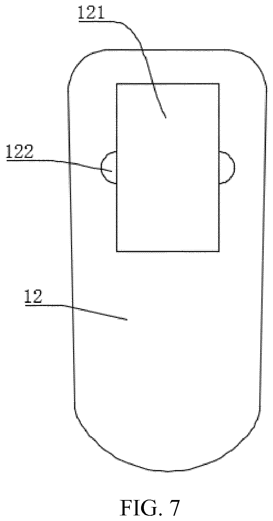

Further, referring to FIGs. 1 to 7, as an alternative embodiment, the shoe sole 12 is provided with a recess 121, and the control module 2 is detachably received in the recess 121. The control module 2 includes a sensor 23 and a controller 24. The sensor 23 is electrically coupled to the controller 24. The sensor 23 collects user data and the controller 24 processes the collected user data.

In the embodiment, since the control module 2 is detachably mounted to the shoe sole 12, when the pedometer shoe 100 needs to be cleaned, the control module 2 can be detached, thereby effectively preventing the control module 2 from water, and avoiding damage to components (such as the sensor 23 and the controller 24) integrated in the control module 2, so that the pedometer shoe 100 has a long service life. At the same time, the detached control module 2 can also be quickly charged and does not affect the pedometer shoe 100 as a shoe for daily use. Moreover, since the sensor 23 can collect user data, and the controller 24 can process the user data, so that the pedometer shoe 100 is multi-functionalized, and can meet diverse market demands.

Preferably, the user data includes but is not limited to step data, altitude data, speed data, acceleration data, foot temperature data, foot pressure data, walking distance data, and so on.

Further, referring to FIGs. 1 to 7, as an alternative embodiment, the shoe sole 12 includes a top surface facing foot and a bottom surface facing the ground, and the recess 121 is defined on the top surface.

In the embodiment, since the recess 121 is defined on the top surface, that is, the control module 2 is mounted on the top surface of the shoe sole 12, thus, the shoe sole 12 can provide protection to the control module 2 during the daily use of the pedometer shoe 100, without adding additional protection.

Further, as an alternative embodiment, the control module 2 fits the inner wall of the recess 121 to prevent the control module 2 from loosening, so that the components in the control module 2 can also be prevented from being damaged by the collision during the movement of the pedometer shoe 100.

Preferably, the width of the recess 121 is slightly less than that of the control module 2 to allow the control module 2 to fit the inner wall of the recess 121.

Preferably, the recess 121 has an inverted trapezoidal shape so that the control module 2 can be easily embedded into the recess 121.

Preferably, the top surface of the shoe sole 12 is further provided with two dimples 122 which are respectively located at two sides of the recess 121, and both of the two dimples 122 communicate with the recess 121. The two dimples 122 can facilitate the user to hold the control module 2 by hand, thereby reducing the difficulty of detaching the control module 2.

Further, referring to FIGs. 1 to 6, as an alternative embodiment, the pedometer shoe 100 further includes a plug 213. The display module 3 is detachably plugged into the control module 2 via the plug 213.

In the embodiment, since the plug 213 is detachably plugged into the control module 2, that is, the connection between the control module 2 and the display module 3 is detachable, therefore, the user can quickly connect or disconnect the control module 2 with the display module 3.

Further, as an alternative embodiment, the plug 213 is fixed in the shoe sole 12, and when the control module 2 is received in the recess 121, the plug 213 is plugged into the control module 2 to electrically connect the control module 2 to the display module 3. When the control module 2 is taken out from the recess 121, the power supply of the display module 3 is cut off.

Further, as an alternative embodiment, the user data includes step data of the user, and the controller 24 of the control module 2 controls the display module 3 to display a corresponding prompt according to the step data. At this time, the pedometer shoe 100 can count steps and display the step count, so that the user can learn his or her real-time exercise amount in time.

Further, referring to FIGs. 1 to 4, as an alternative embodiment, the first flexible circuit board 331 is coupled to the plug 213 through the second flexible circuit board 34. Both the first flexible circuit board 331 and the second flexible circuit board 34 can be freely bent according to the shape of the pedometer shoe 100.

Further, referring to FIGs. 1 to 4, as an alternative embodiment, the third flexible circuit board 36 is coupled to the flexible touch panel 35 and the plug 213. The third flexible circuit board 36 can be freely bent according to the shape of the pedometer shoe 100.

Further, as an alternative embodiment, the plug 213 includes a signal conversion module, and the second flexible circuit board 34 and the third flexible circuit board 36 are both connected to the signal conversion module.

Preferably, the signal conversion module converts a circuit board connection terminal (for example, a gold finger) into a USB connection terminal.

Referring to FIGs. 1 to 6, further, as an alternative embodiment, the control module 2 further includes a housing 21, a circuit board 22, and a battery 25. The circuit board 22 is located in the housing 21, and the controller 24 and the sensor 23 are both electrically connected to the circuit board 22. The battery 25 is configured to supply power for the sensor 23, the controller 24, and the display module 3.

For example, the sensor 23 detects motion data and transmits a data signal to the controller 24 via the circuit board 22. The controller 24 receives and processes the data signal, and forms a control signal. The control signal is transmitted to the display module 3 through the circuit board 22, so that the display module 3 displays a corresponding prompt.

Further, referring to FIGs. 1 to 6, as an alternative embodiment, the control module 2 further includes a first interface 212 mounted on the housing 21, and the first interface 212 is electrically connected to the circuit board 22. The plug 213 is detachably plugged into the first interface 212.

In the embodiment, since the plug 213 can be conveniently plugged into the first interface 212, and the connection between the plug 213 and the first interface 212 is detachable, therefore, the user can quickly connect or disconnect the control module 2 with the display module 3.

Preferably, the first interface 212 is a waterproof interface for improving the waterproof performance of the control module 2.

Further, referring to FIGs. 1 to 6, as an alternative embodiment, a first communication hole 211 is defined on the housing 21 for communicating with an inside and an outside of the housing 21. The first interface 212 is mounted to the first communication hole 211.

Further, referring to FIGs. 1 to 6, as an alternative embodiment, the control module 2 further includes a second interface 215 mounted on the housing 21. When the control module 2 is removed from the recess 121, the second interface 215 is connected to an external power source to charge the battery 25. Of course, the second interface 215 can also connect the pedometer shoe 100 to other components for data transmission.

Further, referring to FIGs. 1 to 6, as an alternative embodiment, the first interface 212 and the second interface 215 are respectively located at opposite ends of the housing 21. For example, a second communication hole opposite to the first communication hole 211 is defined on the housing 21. The second communication hole is configured to communicate with the inside and the outside of the housing 21. The second interface 215 is mounted to the second communication hole.

Preferably, the second interface 215 is a waterproof interface for improving the waterproof performance of the control module 2.

Optionally, the control module 2 further includes a Bluetooth component and/or a GPS component and/or a height sensor and/or a speed sensor, which are electrically connected to the circuit board 22, so that the step shoe 100 has more functions to meet diverse market demands.

Further, referring to FIGs. 1 to 6, as an alternative embodiment, the housing 21 includes a bottom frame 217 and a top cover 218. The bottom frame 217 includes a bottom wall 2171 and a side wall 2172 connected to peripheries of the bottom wall 2171. A sealing groove 2173 is defined on the top of the side wall 2172. The bottom edge of the top cover 218 is provided with a flange 2181. The flange 2181 is embedded into the sealing groove 2173 for sealing the housing 21. At the same time, the bottom frame 217 and the top cover 218 can be locked by a plurality of fasteners 219, so that the housing 21 is sealed firmly. The fasteners 219 include, but are not limited to, screws, rivets, and the like.

Preferably, the housing 21 further includes a waterproof rubber ring 210 located in the sealing groove 2173 for simultaneously abutting the bottom wall of the sealing groove 2173 and the bottom wall of the flange 2181, to further improve the sealing performance of the housing 21.

In the embodiment, the first communication hole 211 and the second communication hole may be defined on two opposite side portions of the side wall 2172.

Preferably, the sensor 23, the controller 24, the battery 25, the circuit board 22, the first interface 212, and the second interface 215 are all secured within the housing 21. Therefore, all components inside the housing 21 are not shaken with respect to the housing 21 during the sway of the pedometer shoe 100, which can effectively prevent all components inside the housing 21 from being injured. For example, the sensor 23 and the controller 24 are both fixed on the circuit board 22, and the circuit board 22, the first interface 212 and the second interface 215 can be fastened to the bottom wall 2171 by the fastener 219, and the battery 25 is tightly held between the circuit board 22 and the bottom wall 2171. Of course, the first interface 212 and the second interface 215 can also be tightly held between the circuit board 22 and the bottom wall 2171.

The embodiments of the present disclosure have been described in detail above, and the principles and implementations of the present disclosure are described herein by using specific examples. The description of the above embodiments is merely for assisting in understand the method of the present disclosure and its core ideas. At the same time, for those skilled in the art, according to the idea of the present disclosure, there will be changes in the specific embodiments and application scopes. In conclusion, the content of the specification should not be construed as limiting the present disclosure.

## Claims

1. A pedometer shoe comprising:
a shoe body, comprising a vamp and a shoe sole which are connected with each other;
a control module, arranged in the shoe sole and sending out signals according to step counts; and
a display module attached to the vamp, the display module comprising a display area, and receiving the signals and displaying corresponding prompts.

2. The pedometer shoe of claim 1, wherein the display module displays different colors according to different step counts.

3. The pedometer shoe of claim 1, wherein the display module comprises a plurality of the display areas, and the display areas are lighted up one by one according to different step counts.

4. The pedometer shoe of claim 3, wherein heights of the plurality of display areas arranged from a shoe upper toward a shoe toe of the vamp are gradually decreased.

5. The pedometer shoe of any of claims 1 to 4, wherein the display module comprises:
a light guide plate, comprising a light exiting surface and a reflecting surface which are opposite to each other, and a light incident surface connected between the light exiting surface and the reflecting surface, wherein the reflecting surface is attached to the vamp, to cause the light exiting surface facing an outside of the shoe body; and
a light source facing the light incident surface, wherein light emitted from the light source passes through the light incident surface and exits from the light exiting surface.

6. The pedometer shoe of claim 5, wherein the light guide plate is made of flexible material.

7. The pedometer shoe of claim 5, wherein the display module further comprises a flexible touch panel, wherein the flexible touch panel generates a touch signal according to a touch operation, and the control module adjusts the prompt displayed by the display module according to the touch signal.

8. The pedometer shoe of claim 7, wherein the flexible touch panel generates the touch signal according to a long-pressing touch operation, and the control module adjusts the color of the display module according to the touch signal.

9. The pedometer shoe of claim 7, wherein the flexible touch panel generates the touch signal according to a sliding touch operation, and the control module adjusts brightness of the display module according to the touch signal.

10. The pedometer shoe of claim 7, wherein the flexible touch panel is attached to the light exiting surface.

11. The pedometer shoe of claim 7, wherein the flexible touch panel comprises a touch area and a non-touch response area, the touch area is arranged opposite to the display area, and the non-touch response area is located outside a range in which the touch area is located.

12. The pedometer shoe of claim 11, wherein the flexible touch panel comprises a plurality of the touch areas and a plurality of the non-touch response areas, and the touch areas and the non-touch response areas are alternately arranged.

13. The pedometer shoe of claim 7, wherein the display module further comprises a protective film attached to the flexible touch panel, the flexible touch panel being sandwiched between the protective film and the light guide plate.

14. The pedometer shoe of claim 13, wherein the protective film comprises the display area and an opaque area, the opaque area being located outside a range in which the display area is located.

15. The pedometer shoe of claim 14, wherein the protective film comprises a plurality of the display areas and a plurality of the opaque areas, and the opaque areas and the display areas are alternately arranged.

16. The pedometer shoe of claim 7, wherein the display module further comprises a uniform light film arranged between the flexible touch panel and the light exiting surface.

17. The pedometer shoe of claim 7, wherein the display module further comprises a first flexible circuit board, wherein the light source is mounted on the first flexible circuit board.

18. The pedometer shoe of claim 17, wherein the first flexible circuit board is bent around a shoe upper of the vamp.

19. The pedometer shoe of claim 17, wherein the first flexible circuit board is electrically coupled to the control module through a second flexible circuit board, and the flexible touch panel is electrically coupled to the control module through a third flexible circuit.

20. The pedometer shoe of any of claims 1 to 4, wherein the display module is bent around a shoe upper of the vamp and is attached to two opposite sides of the vamp.
